# EUROPEAN PATENT SPECIFICATION

(11) **EP 2 190 424 B1**
(45) Date of publication and mention of the grant of the patent: **20.03.2013**
(21) Application number: 08804813.7
(22) Date of filing: 26.09.2008
(51) Int. Cl.: A61K 31/015, A61K 31/045, A61K 31/121, A61K 31/122, A61K 31/19, A61K 31/22, A61P 17/02, A61P 17/04, A61P 17/06, A61Q 19/00, A61K 8/34

(54) **USE OF PERILLYL ALCOHOL TO INCREASE TISSUE REPAIR**
VERWENDUNG DES PERILLYLALKOHOLS ZUR VERSTÄRKUNG EINER GEWEBEREPARATUR
UTILISATION DE L'ALCOOL PERILLYLIQUE POUR INDUIRE LA RÉPARATION TISSULAIRE

(30) Priority: 26.09.2007 EP 07291145
(43) Date of publication of application: 02.06.2010
(73) Proprietor: AISA THERAPEUTICS, 91058 Evry Cedex (FR)
(72) Inventor: D'ALESSIO, Patrizia, F-75007 Paris (FR)
(74) Representative: Jacobson, Claude
(86) International application number: PCT/EP2008/062938
(87) International publication number: WO 2009/040420

(56) References cited:
- WO-A-01/78706
- WO-A-02/15920
- WO-A-2004/004650
- WO-A-2004/058315
- WO-A-2005/063213
- WO-A-2005/105074
- WO-A-2006/071849
- WO-A-2006/134013
- DE-A1- 19 644 422
- FR-A- 2 790 667
- JP-A- 4 334 319
- JP-A- 11 035 455
- US-A- 5 961 997
- US-A1- 2005 079 236
- US-A1- 2006 051 432
- US-A1- 2007 009 474
- DATABASE WPI Week 200517 Thomson Scientific, London, GB; AN 2005-152785 XP002465256 & CN 1 541 666 A (SHENZHEN EAST MATERIA SCI & TECHNOLOGY D) 3 November 2004 (2004-11-03)
- DATABASE WPI Week 199415 Thomson Scientific, London, GB; AN 1994-121179 XP002465257 & JP 06 065087 A (KANEI KK) 8 March 1994 (1994-03-08)
- PANAHI Y ET AL: "Phenol and menthol in the treatment of chronic skin lesions following mustard gas exposure" SMJ SINGAPORE MEDICAL JOURNAL, vol. 48, no. 5, May 2007 (2007-05), pages 392-395, XP007903865 ISSN: 0037-5675
- DATABASE WPI Week 199539 Thomson Scientific, London, GB; AN 1995-300910 XP002465258 & RU 2 029 544 C1 (GUSEV V V) 27 February 1995 (1995-02-27)
- DATABASE WPI Week 200411 Thomson Scientific, London, GB; AN 2004-102443 XP002465259 & JP 2003 153945 A (FUKUMOTO Y) 27 May 2003 (2003-05-27)
- DATABASE WPI Week 200405 Thomson Scientific, London, GB; AN 2004-046818 XP002465260 & JP 2003 261413 A (KURATA K) 16 September 2003 (2003-09-16)

## Description

The invention relates to the use of perillyl alcohol to increase biological tissue repair in cosmetic and therapeutic indications.

Tissue repair includes the processes reaching to the recovery of a lesion, with or without relapse, by tissue regeneration or healing.

Intrinsic tissue regeneration is the body's regular maintenance in which millions of cells constantly produce tissue remodelling and restoration of tissue functions. It begins with stem cells. Biochemical signals draw the stem cells to sites where growth factors and cytokines have created an environment for regeneration.

Scar tissue is different from regenerated tissue. When an injury occurs, the body's first reaction is haemostasis when fibrin and inflammatory cytokines form a blood clot or provisional scaffold. More inflammatory cells arrive, remodelling the clot into scar tissue. Collagen in scar tissue is abnormally aligned and has little elastin. Unlike regenerated tissue, scar tissue is different-and less perfect- than the surrounding tissue it replaces.

Terpenes are a large and varied class of hydrocarbons, produced primarily by a wide variety of plants, particularly conifers, though also by some insects such as swallowtail butterflies, which emit terpenes from their osmeterium. They are the major components of resin, and of turpentine produced from resin. The name "terpene" is derived from the word "turpentine". When terpenes are modified chemically, such as by oxidation or rearrangement of the carbon skeleton, the resulting compounds are generally referred to as terpenoids. Some authors will use the term terpene to include all terpenoids. Terpenes and terpenoids are the primary constituents of the essential oils of many types of plants and flowers. Essential oils are used widely as natural flavor additives for food, as fragrances in perfumery, in aromatherapy, and in traditional and alternative medicines. Synthetic variations and derivatives of natural terpenes and terpenoids also greatly expand the variety of aromas used in perfumery and flavors used in food additives.

Terpenes are derived biosynthetically from units of isoprene, which has the molecular formula C₅H₈. The basic molecular formulas of terpenes are multiples of that, (C₅H₈)ₙ where n is the number of linked isoprene units. Isoprene itself does not undergo the building process, but rather activated forms, isopentenyl pyrophosphate (IPP or also isopentenyl diphosphate) and dimethylallyl pyrophosphate (DMAPP or also dimethylallyl diphosphate), are the components in the biosynthetic pathway. As chains of isoprene units are built up, the resulting terpenes are classified sequentially by size as hemiterpenes, monoterpenes, sesquiterpenes, diterpenes, sesterterpenes, triterpenes, and tetraterpenes.

Monoterpenes consist of two isoprene units and have the molecular formula C₁₀H₁₆. Examples of monoterpenes are: geraniol and limonene. Monoterpenes occur in monocyclic, bicyclic, and acyclic forms and are either simple or modified hydrocarbons. They are a class of isoprenoid molecules derived from the anabolism of acetate by the mevalonic acid branch biosynthetic pathways of plants.
D-Limonene ((4R)-1-methyl-4-isopropenylcyclohex-1-ene), a major component of orange peel oil and the prototype of monoterpenes in carcinogenesis studies, is formed by the cyclization of the 10-carbon isoprene intermediate geranylpyrophosphate.

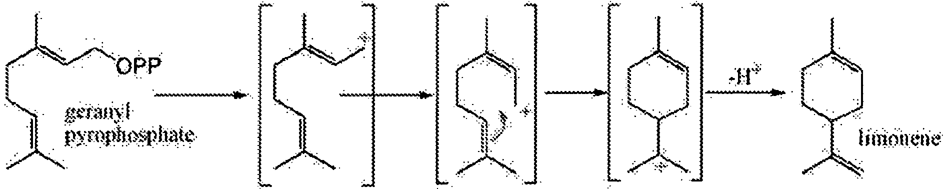

D-Limonene and its derived metabolites have been shown to possess cancer chemotherapeutic and chemopreventive efficacy in various preclinical model systems.

Crowell PL *et al.* identified plasma metabolites of limonene in blood of seven healthy human volunteers having ingested 100 mg/kg limonene in a custard. On-line capillary gas chromatography/mass spectrometry analysis indicated that at least five compounds were present at 4 h after ingestion. Two major peaks were identified as the rat limonene metabolites dihydroperillic acid and perillic acid, and two minor peaks were found to be the respective methyl esters of these acids (Crowell PL *et al.,* 1994).

Although R- and S-limonene are only weak inhibitors of the isoprenylation enzymes, their major metabolites, perillic acid and perillyl alcohol, are more potent inhibitors, with IC50 values in the low mM range. The metabolites possess greater activity towards the geranylgeranyltransferase type I enzyme than farnesyltransferase (Hardcastle IR *et al.,* 1999).

D-Limonene has a pronounced chemotherapeutic activity and minimal toxicity in preclinical studies. A phase I clinical trial to assess toxicity, the maximum tolerated dose (MTD) and pharmacokinetics in patients with advanced cancer was followed by a limited phase II evaluation in breast cancer. D-Limonene is well tolerated in cancer patients at doses which may have clinical activity (Vigushin DM *et al.,* 1998).

Chemical compounds derived from plants used in traditional medicine to cure disease, are an important source for the development of new active pharmaceutical molecules. Using such a strategy, the inventors have previously identified several monoterpenes able to specifically prevent or treat the senescence of human vascular endothelial cells induced by repeated inflammatory episodes (WO 2005/105074).

The inflammatory response is the first vitally necessary mechanism of tissue repair of injured tissues. It is characterized by immediate events to prevent loss of red blood cells, an acute response to remove damaged tissues and establish a vascular supply to support repair and a tissue repair phase.

Nonsteroidal anti-inflammatory drugs, like ibuprofen, an anti-inflammatory molecule on the market, impede the last stage of inflammation process, i.e. tissue repair, by virtue of retarding inflammation. Anti-inflammatory agents whose principal effect is to diminish granulocytic inflammatory reaction also have healing-depressant propensity (Lee KH *et al.,* 1968).

Nonsteroidal anti-inflammatory drugs that decrease breaking strength in incision wounds were found to have variable effects on wound contraction, and epithelialization (Rao CM. *et al.,* 1988; Kumar A. et *al.,* 1988).

Ibuprofen was reported to reduce the breaking strength of the repaired extensor tendon, wound contraction, and epithelialization (Dvivedi S. et *al.,* 1997, Kulick MI. *et al.,* 1986; Dong Y-L. *et al.,* 1993).

The present inventors have now unexpectedly shown that limonene (a reference compound) and perillyl alcohol induce biological tissue repair despite the anti-inflammatory effect of limonene previously observed by the present inventors (W02005/105074).

First, the present inventors have now unexpectedly observed in a rat colon inflammation model that the anti-inflammatory effect of limonene does not impede tissue repair.

Second, the present inventors have shown that limonene and perillyl alcohol are beneficial against chronic skin inflammation disorders like atopic dermatitis.

Third, the present inventors have shown the beneficial effect of limonene and perillyl alcohol in a classical model of healing.

In order to appreciate the effects of limonene on tissue repair, 30 Wistar HsdBrIHan female rats were used in a colitis rat model. Colon inflammation was induced by a single rectal administration of an acid solution of sulfonic 2,5,6 - trinitrobenzene (TNBS, Fluka, France) and the anti-inflammatory effect of limonene on tissue repair given by oral administration was studied. Average macroscopic and microscopic scores of the colons sampled 6 days after the induction of colon inflammation were compared. The results showed that limonene, compared with ibuprofen, induced post-inflammatory tissue repair, despite its anti-inflammatory effect. Moreover, it decreased the circulating TNF-α level at a concentration five times inferior to the concentrations of Ibuprofen, used as a reference molecule.

In a model of chronic skin inflammation, the inventors have also shown a significant effect of limonene and perillyl alcohol on skin repair and pro-inflammatory cytokines levels. Chronic skin inflammation has been induced in 24 female mice hairless Skh-1 by dorsal daily applications of Phorbol 12-myristate-13-acetate (TPA) during seven days (Stanley P.L. *et al.,* 1991).

In a second skin model, the influence of limonene and perillyl alcohol on healing process was studied in hairless Skh-1 mice submitted to a scarification of the skin on each flank. Com oil alone, limonene in solution in corn oil or perillyl alcohol in solution in corn oil were administered daily by topic application on each site of scarification at the dose of 10 mg/kg/day for 8 days. The wound healing process gave better results with daily applications of perillyl alcohol in comparison with limonene and much better results in comparison with the control.

### Use of perillyl alcohol to increase tissue repair

Thus, the present invention relates to the use of perillyl alcohol to increase biological tissue repair in cosmetic and therapeutic indications.

Preferably, tissue repair includes tissue regeneration and healing.

In one embodiment, perillyl alcohol is used according to the invention to increase biological tissue regeneration.

Preferably, perillyl alcohol is used according to the invention to increase biological tissue regeneration *in vitro.*

In the context of the present invention, a "tissue" means a collection of interconnected cells.

In the context of the present invention, a "biological tissue" means a tissue of human or animal origin and includes *in vivo* tissues and *in vitro* or ex *vivo* cultured tissues.

Examples of *in vitro* or ex *vivo* cultured tissues include cultured cells as fibroblasts and *in vitro* skin models which associate different cell types found in skin.

Examples of *in vivo* biological tissues include skin and mucosae.

In one embodiment, skin means scalp.

In the context of the present invention, a "biological tissue repair" means tissue regeneration and/or healing of a biological tissue showing injuries and/or defects and/or disease symptoms.

Biological tissue injuries include burns, cuts, burning relapses, wounds and relapses of extreme weather conditions including excessive cold or heat.

Biological tissue diseases include skin tissue diseases as atopic dermatitis, seborrhoic keratitis, epidermolysis bullosa acquisita, psoriasis, skin alterations in lupus erythematosus, dermatomyositis, scleroderma, chronic acne, chronic cellulites, pruritus and abnormal or defective scar formation as in diabetes.

Biological tissue defects include skin tissue defects such as acne pimples, cellulites, scars, sun and UV induced premature skin senescence, unesthetic skin defaults due to the aging process including scars and stains, wrinkles and squamous features, stretch marks, aging marks, sun and UV induced premature skin senescence, rosacea and unesthetisms of post-traumatic relapses.

The use of perillyl alcohol to increase biological tissue repair may be cosmetic or therapeutic.

For such a use, perillyl alcohol may be administered by a systemic route or by a topic route. Preferably, the systemic route is selected from the group consisting of oral, lingual, sublingual, muscular and intravenous routes. More preferably, the systemic route is the oral route.

Preferably, a cosmetic use of perillyl alcohol may be in the form of a cosmetic formulation used topically or in the form of a food complement used orally.

### Cosmetic use

In another embodiment, the invention relates to the cosmetic use of perillyl alcohol to increase biological tissue repair of skin or a mucosa in an individual.

Preferably, the invention relates to the use of perillyl alcohol orally or topically to increase biological tissue repair of skin or a mucosa in an individual.

Preferably, the invention relates to the cosmetic use of perillyl alcohol to increase tissue regeneration of skin or a mucosa in an individual.

Skin includes epidermis and dermis. Epidermis is the outermost layer of the skin. The main type of cells which make up the epidermis are keratinocytes, melanocytes, Langerhans cells and Merkels cells. The dermis is the layer of skin beneath the epidermis that consists of connective tissue.

Examples of mucosae include peri-oral mucosa, buccal mucosa, gastric and colon mucosa, genital mucosa.

As used herein, the term "individual" denotes a human, a pet, a laboratory animal or a farm animal, more preferably a bird or a mammal, such as a rodent, a feline, an equine, a bovine, a caprine, a canine, a porcine and a primate. Preferably, an individual according to the invention is a human, a cat or a dog.

"Topically" means applied to the skin or a mucosa.

In a further embodiment, the invention relates to a method for preventing or treating non-pathological conditions of skin or mucosa defects.

Preferably, a method for preventing or treating non-pathological conditions of skin or mucosa defects according to the invention comprises the application of a cosmetic formulation comprising perillyl alcohol, in one dose or in repeated doses and at specified intervals of time, on said skin or mucosa defect.

In the frame of methods for preventing or treating non-pathological conditions of skin or mucosa defects according to the invention, the administration regimen may be for instance for a period of more than 4 weeks, or more than 8 weeks.

Preferably, in the frame of methods for preventing or treating non-pathological conditions of skin or mucosa defects according to the invention, the range of dose of monoterpene may be between 0.1 mg/kg/day and 100 mg/kg/day. More preferably, the dose range is between 1 mg/kg/day and 100 mg/kg/day. Most preferably, the dose range is between 10 mg/kg/day and 50 mg/kg/day.

Preferably, the cosmetic use of perillyl alcohol to increase biological tissue repair of skin or mucosa in an individual may be in a form selected in the group consisting of patch, capsule, pill, cream, paste (toothpaste), detergent such as shampoo and dermo-soap, bubble bath or bath salts, aqueous, alcoholic or oily lotion, gel, low ozone spray.

Preferably, the monoterpene perillyl alcohol is entrapped in liposomes or any other chemical or mechanical trapping system or device permitting a delayed effect.

The cosmetic use of perillyl alcohol is preferably carried out topically by the application of a cosmetic formulation on skin or a mucosa.

Cosmetic formulations may include, besides perillyl alcohol, other active principles and one or more cosmetically acceptable carriers.

As used herein, "cosmetically acceptable carriers", means a carrier to be used in contact with the different superficial parts of the human body, for example epidermis, without toxicity, irritation, undue allergic response or other untoward reaction.

Such cosmetic formulations are generally in form of lotion, emulsion, cream, gel, shampoo or patch.

The one skilled in the art will be able to specify the supplementary components to be carried out in cosmetic formulations according to the type of cosmetic formulation to be obtained.

Preferably, the invention relates to the use of perillyl alcohol to increase skin or mucosa tissue repair on a skin or mucosa defect, said defect being preferably selected from the group consisting of unesthetic skin defaults such as cellulites, scars, sun and UV induced premature skin senescence, unesthetic skin defaults due to the aging process including scars and stains, wrinkles and squamous features, stretch marks, aging marks, sun and UV induced premature skin senescence and unesthetisms of post-traumatic relapses.

### Pharmaceutical use

A further object of the present invention relates to the use of perillyl alcohol for the manufacture of a pharmaceutical composition intended to prevent or treat a biological tissue injury in an individual.

A further object of the present invention relates to the use of perillyl alcohol for the manufacture of a pharmaceutical composition intended to prevent or treat a biological tissue disease.

Preferably, said biological tissue injury or disease is a skin injury or disease.

In a preferred embodiment, said prevention or treatment concerns a human, a pet, a laboratory animal or a farm animal. More preferably, said prevention, alleviation or treatment concerns an individual selected in the group consisting of a bird or a mammal, such as a rodent, a feline, an equine, a bovine, a caprine, a canine, a porcine and a primate. Most preferably, said prevention, alleviation or treatment concerns a human, a cat or a dog.

Preferably, said pharmaceutical composition is used topically or systemically, more preferably orally.

Such pharmaceutical compositions comprise perillyl alcohol as active ingredient and pharmaceutically acceptable carriers.

"Pharmaceutically acceptable" refers to molecular entities and compositions that do not produce an adverse, allergic or other untoward reaction when administered to an animal, or a human, as appropriate.

As used herein, "pharmaceutically acceptable carrier" includes any and all solvents, dispersion media, coatings, antibacterial and antifungal agents, isotonic and absorption delaying agents and the like. The use of such media and agents for pharmaceutical active substances is well known in the art. Except insofar as any conventional media or agent is incompatible with the monoterpene used according to the invention, its use in the pharmaceutical compositions, in the medicaments, or for implementing the methods for preventing or treating a biological tissue injury in an individual according to the invention is contemplated. Supplementary active ingredients can also be incorporated into the pharmaceutical compositions.

In the context of the invention, the terms "to prevent", "preventing" or "prevention" means to allow avoiding a tissue injury.

In the context of the invention, the terms "to treat", "treating" or "treatment", means reversing, alleviating, or inhibiting a tissue injury.

In the context of the invention, a tissue disease is preferably a skin disease and may include skin tissue diseases such as atopic dermatitis, seborrhoic keratitis, epidermolysis bullosa acquisita, psoriasis, skin alterations in lupus erythematosus, dermatomyositis, scleroderma, chronic acne, chronic cellulites, pruritus and abnormal or defective scar formation as in diabetes.

In the context of the invention, a tissue injury is preferably a skin injury and may include burns, cuts, relapses of extreme weather conditions including excessive cold or heat, burning relapses and wounds.

In a further embodiment, the invention relates to perillyl alcohol for use in a method for preventing or treating a biological tissue injury or disease in an individual.

Preferably, a method for preventing or treating a biological tissue injury or disease in an individual according to the invention comprises the administration of a pharmaceutical composition comprising perillyl alcohol, in one dose or in repeated doses and at specified intervals of time.

In the frame of methods for preventing or treating a biological tissue injury or disease in an individual according to the invention, the administration regimen may be by a systemic or topic way, preferably by a topic way.

In the frame of methods for preventing or treating a biological tissue injury or disease in an individual according to the invention, the administration regimen may be for instance for a period of less than 6 weeks, or less than 8 weeks.

Preferably, in the frame of methods for preventing or treating a biological tissue injury or disease in an individual according to the invention, the range of dose of monoterpene may be between 0.1 mg/kg/day and 100 mg/kg/day. More preferably, the dose range is between 1 mg/kg/day and 100 mg/kg/day. Most preferably, the dose range is between 10 mg/kg/day and 50 mg/kg/day. More preferably, the dose range is between 10 mg/kg/day and 50 mg/kg/day.

The pharmaceutical use of perillyl alcohol is preferably carried out topically by the application of a pharmaceutical formulation on skin or a mucosa.

Preferably, a pharmaceutical composition comprising perillyl alcohol for use in the prevention or treatment of a biological tissue injury in an individual may be in a form selected in the group consisting of patch, capsule, pill, ointment and wound-dressing.

The invention will be further illustrated in view of the following figures and examples.

### FIGURES

Figure 1 shows the rats average weights evolution (Mean ± ESM).
Figure 2 shows the average serum levels of TNF-α (pg/ml) (Mean ± ESM).
   U Test Mann-Whitney:
   - * P < 0.05 and ** P< 0.01 (vs. "Control") ;
   - ^{#} P < 0.05 (vs. "TNBS/Control").
Figure 3 shows the average macroscopic scores of the rats colons of the 5 groups of treatment (Mean ± ESM).
   U Test Mann-Whitney:
   - ** P < 0.01 (vs. "Control") ;
   - ^{#} P < 0.05 and ^{##} P < 0.01 (vs. "TNBS/Control") ;
   - ° P < 0.05 (vs. "TNBS/Limonene 100").
Figure 4 shows the average colon lengths (cm) of the rats of the 5 groups of treatment.
   (Mean ± ESM) U Test Mann-Whitney :
   - ** P < 0.01 (vs. "Control");
   - ^{#} P < 0.05 (vs. "TNBS/Control").
Figure 5 shows the mice average weights evolution (Mean ± ESM).
Figure 6 shows the average macroscopic scores of skin inflammation of the mice of the 4 groups of treatment.
Figure 7 shows the average microscopic scores of skin inflammation of the mice of the 4 groups of treatment.
Figure 8 shows the average serum levels of IL-1β (pg/ml) of the mice of the 4 groups of treatment before killing.
Figure 9 shows the average serum levels of IL-6 (pg/ml) of the mice of the 4 groups of treatment before killing.

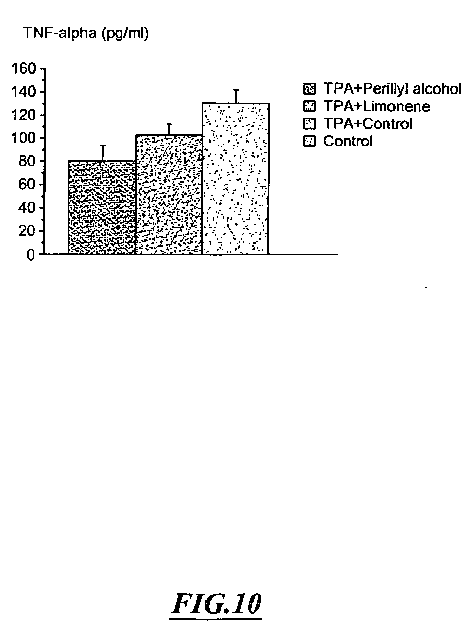

Figure 10 shows the average serum levels of IFN-γ (pg/ml) of the mice of the 4 groups of treatment before killing.

### EXAMPLES

Limonene is to be considered as a reference example

### Example 1 : Preventive anti-inflammatory effects of limonene in a rat colitis model

### MATERIAL AND METHODS

### 1 - Animals

Thirty female rats Wistar HsdBrIHan EOPS (Ganat, France) with an average weight of 175-200 g were used.

### 2 - Assayed products

Limonene ((R)-(+)-Limonene, MW = 136.23, purity = 97%) and ibuprofen ((S)-(+)-Ibuprofen, MW = 206.28, purity = 100%) from SIGMA-ALDRICH (Saint-Quentin Fallavier, France) were used.
Limonene and Ibuprofen were prepared in maize oil extemporarily every day of treatment.

### 3 - Preventive anti-inflammatory effects of Limonene on colon inflammation

The 30 rats were weighed, marked and shared in 5 groups of 6 (n = 6) :
- "control" group = no colon inflammation and daily oral treatment with maize oil ;
- "TNBS control" group = induced colon inflammation and daily oral treatment with maize oil ;
- "TNBS + Limonene 10" group = induced colon inflammation and daily oral treatment with Limonene at 10 mg/kg ;
- "TNBS + Limonene 100" group = induced colon inflammation and daily oral treatment with Limonene at 100 mg/kg ;
- "TNBS + Ibuprofen" group = induced colon inflammation and daily oral treatment with Ibuprofen at 50 mg/kg.

### 3.1- Colon inflammation induction

Colon inflammation was induced by a unique rectal administration of a 2,4,6-trinitrobenzen-sulfonic acid solution (TNBS, Fluka, France). TNBS was dissolved in 40° alcohol at a concentration of 50 mg/ml. Animals were anaesthetised intraperitoneally by 2 mg/kg of Calmivet (Vetoquinol, Lure, France) and 50 mg/kg of Ketamine (Ketamine 1000, Virbac, Carros, France). 0.4 ml of the TNBS solution was administered rectally and the rats were maintained during at least 30 minutes to avoid the releasing of the TNBS.

### 3.2 - Products administration (Table 1)

Limonene was administered daily and orally at 10 and 100 mg/kg, during 3 days before and 5 days after the colon inflammation induction.
Ibuprofen, used as control, was administered daily and orally at 50 mg/kg, during 3 days before and 5 days after the colon inflammation induction.

**Table 1 : summary of the treatment conditions**

| **Groups** | **Rats Nr** | **Colon inflammation induction** | | **Treatment days** |
|---|---|---|---|---|
| | | **Product** | **Induction day** | |
| **Control** | 6 | Maize oil | J₁ | **J₋₂ to J₅** |
| **TNBS control** | 6 | TNBS | J₁ | |
| **TNBS + Limonene 10** | 6 | TNBS | J₁ | |
| **TNBS + Limonene 100** | 6 | TNBS | J₁ | |
| **TNBS + Ibuprofen** | 6 | TNBS | J₁ | |

### 4 - Statistics

Kruskal-Wallis test followed possibly by the Mann-Whitney test was used to compare the different studied variables of the treated groups by comparison with the groups "Control" and "TNBS/Control". Significativity threshold was settled at P < 0.05.

Statistics were carried out with the Statview 5 software (SAS, Institute Inc., USA).

### RESULTS

### 1. - Animal weight progress

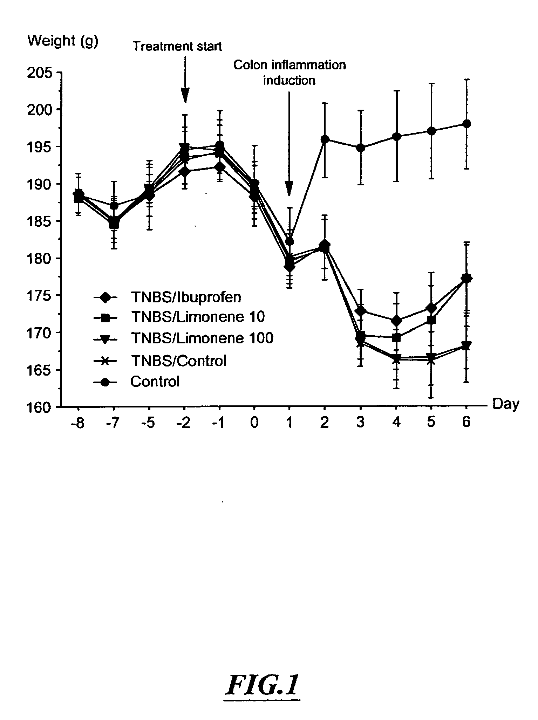

Figure 1 shows the average weight progress of the animals of the 5 groups during the assay.

Kruskal-Wallis Test (H_{(ddl=4)} = 0.381 ; *P* = 0.984) did not show significant heterogeneity among the average weights of the rats of the 5 groups of treatments before the treatment at J-2.

At J1, just before the rectally TNBS administration and after a 48 hour-fasting period (J-1 to J1), Kruskal-Wallis test (H_{(ddl=4)} = 0.308 ; *P* = 0.989) did not show significant heterogeneity among the average weights of the rats of the 5 groups of treatments.

At J6, Kruskal-Wallis test (H_{(ddl=4)} = 14.415 ; *P* = 0.0061) showed a significant heterogeneity among the average weights of the rats of the 5 groups of treatments. U test of Mann-Whitney showed that the rats average weights of the groups "TNBS/control", "TNBS/Limonene 10", "TNBS/Limonene 100" and "TNBS/Ibuprofen" were significantly lower than those of the rats of the group "Control". The rats average weights of the groups "TNBS/Limonene 10", "TNBS/Limonene 100" and "TNBS/Ibuprofen" were not significantly different from those of the rats of the group "TNBS/Control". The rats average weights of the groups "TNBS/Limonene 10", "TNBS/Limonene 100" and "TNBS/Ibuprofen" were not significantly different.

### 2 - Colon macroscopic scores

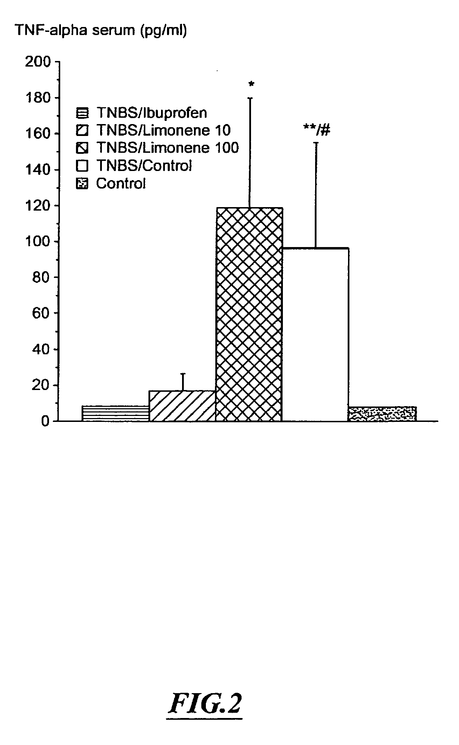

Figure 2 and Table 2 show the average macroscopic scores of the colons of the animals of the 5 groups of treatment removed 6 days after the colon inflammation induction.

Kruskal-Wallis test (H_{(ddl=4)} = 21.925 ; P = 0.0002) showed a significant heterogeneity among the average macroscopic scores of the colons of the rats of the 5 groups of treatment. U test of Mann-Whitney showed that the average macroscopic scores of the colons of the rats of the groups "TNBS/Control", "TNBS/Limonene 10", "TNBS/Limonene 100" and "TNBS/Ibuprofen" were significantly higher than those of the rats of the group "Control". The average macroscopic scores of the colons of the rats of the groups "TNBS/Limonene 10" and "TNBS/Ibuprofen" were significantly lower than those of the rats of the groups "TNBS/Control" and "TNBS/Limonene 100" (z = 2.03 ; P = 0.0427 and z = 2.41 ; P = 0.0159, respectively). The average macroscopic scores of the colons of the rats of the groups "TNBS/Limonene 10" and "TNBS/Ibuprofen" were not significantly different.

**Table 2**

| **Treatment** | **Control (n = 6)** | **TNBS/ Control (n = 6)** | **TNBS/ Limonene 10 (n = 6)** | **TNBS/ Limonene 100 (n = 6)** | **TNBS/ Ibuprofen (n = 6)** |
|---|---|---|---|---|---|
| **Mean ± ESM** | 0.0 ± 0.0 | 10.7 ± 2.0 | 6.5 ± 1.3 | 10.7 ± 2.7 | 4.7 ± 2.7 |
| **Kruskal-Wallis Test** | | | | | |
| **H_{(ddl=4)}** = **21.925 ;** *P* **= 0.0002** | | z = 3.08 | z = 3.09 | z = 3.08 | z = 3.08 |
| **U Test of Mann-Whitney (vs. Control)** | | *P* = 0.0021 | *P* = 0.0020 | *P* = 0.0021 | *P* = 0.0021 |
| **U Test Mann-Whitney** (vs. TNBS/Control) | | | z = 2.50 | z = 0.33 | z = 2.59 |
| | | | *P* = 0.0124 | N.S. | *P* = 0.0097 |

### 3 - Colon length

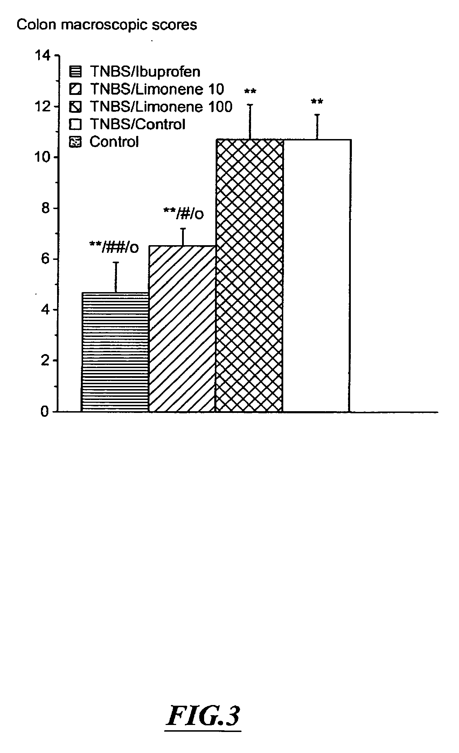

Figure 3 shows the average lengths of the colons of the animals of the 5 groups of treatment removed 6 days after the colon inflammation induction.

Kruskal-Wallis test (H_{(ddl=4)} = 15.827 ; *P* = 0.0033) showed a significant heterogeneity among the average lengths of the colons of the rats of the 5 groups of treatment. U test of Mann-Whitney showed that the average lengths of the colons of the rats of the groups "TNBS/Control", "TNBS/Limonene 10", "TNBS/Limonene 100" and "TNBS/Ibuprofen" were significantly higher than those of the rats of group "Control". The average lengths of the colons of the rats of the groups "TNBS/Limonene 10", "TNBS/Limonene 100" and "TNBS/Ibuprofen" were significantly lower than those of the rats of group "TNBS/Control". The average lengths of the colons of the rats of the groups "TNBS/Limonene 10", "TNBS/Limonene 100" and "TNBS/Ibuprofen" were not significantly different.

### 4 - Histopathological analysis and microscopic scores of the colons

Histopathological analysis of the colons of the animals of the 5 groups of treatment removed 6 days after the colon inflammation induction were carried out on 9 cut layouts for one colon.

Colons of the group "Control" showed a normal colic wall, without oedema, inflammatory infiltrate, necrosis, epithelial atrophy nor dysplasy, corresponding to a microscopic score of 0.

Colons of the group "TNBS/Control" showed in general a colic wall extremely reworked with acute suppurated necrotic and multifocal inflammatory lesions corresponding to a microscopic score of 4.

Colons of the group "TNBS/Limonene 10" showed in general a colic wall reworked with mild multifocal lesions of ulcerous non specific colitis corresponding to a microscopic score of 2.

Colons of the group "TNBS/Limonene 100" showed in general a colic wall extremely reworked with acute suppurated necrotic and multifocal inflammatory lesions corresponding to a microscopic score of 4.

Colons of the group "TNBS/Ibuprofen" showed for one half a colic wall with a mild bifocal lesion of ulcerous non specific colitis corresponding to a microscopic score of 1 and for the other half a colic wall extremely reworked with acute suppurated necrotic and multifocal inflammatory lesions corresponding to a microscopic score of 4.

### Example 2 : Preventive anti-inflammatory effects of limonene and perillyl alcohol in a mouse chronic skin inflammation model

### MATERIAL AND METHODS

### 1 - Animals

24 female mice hairless Skh-1 with an average weight of 20-25 g were used.

The 24 mice were marked and shared in 4 groups of 6 (n = 6) :
- "control" group = no induced skin inflammation and daily topical treatment during 10 days with carrier;
- "TPA+control" group = induced skin inflammation and daily topical treatment during 10 days with carrier ;
- "TPA+Limonene" group = induced skin inflammation and daily topical treatment during 10 days with limonene 10 mg/kg/day;
- "TPA+Perillyl alcohol" group = induced skin inflammation and daily topical treatment during 10 days with perillyl alcohol 10 mg/kg/day;

### 2 - Induction of skin inflammation

Phorbol 12-myristate-13-acetate (TPA, MW = 616.83, purity = at least 98%) was stored before use according to the manufacturer's instructions (SIGMA-ALDRICH, Saint-Quentin Fallavier, France).

100 µl of a TPA solution were applied daily on the dorsal surfaces of mice during seven days (from J₄ to J₁₀) at a concentration of 0.2 mg/ml.

### 3 -Assayed products and treatment conditions

Limonene ((R)-(+)-Limonene, MW = 136.23, purity = 97%) from SIGMA-ALDRICH (Saint-Quentin Fallavier, France) was used and stored according to SIGMA-FLUKA instructions.

Perillyl alcohol ((S)-4-Isopropenyl-1-cyclohexenylmethanol ; (S)-p-Mentha-1,8-dien-7-ol, MW = 152.23, purity = 98%) from SIGMA-ALDRICH (Saint-Quentin Fallavier, France) was used and stored according to SIGMA-FLUKA instructions. Limonene and Perillyl alcohol were prepared in maize oil extemporarily every day of treatment.

The daily topical treatment begun 3 days before the induction of skin inflammation (=J₁) and was continued 7 days after the induction of skin inflammation (=J₁₀). From J₄ to J₁₀, the assayed products were applied 30 minutes before the application of TPA on the same skin area.

### 4 - Serum sampling

The mice were anaesthetized by intraperitoneal injection of a mixture of Ketamine (Ketamine 1000, Virbac, Carros, France) and Xylazine (Rompun 2%, Bayer Healthcare, Kiel, Allemagne).(2/3-1/3 vol/vol) at a dosis of 8 ml/kg. Blood (1 ml) was punctured intracardiacally after the last treatment. Blood samples were stored at +4°C during 20 to 30 minutes then centrifuged at 1500 g for 15 minutes. Serums were removed, freezed at -20°C and stored at -80°C until pro-inflammatory cytokines dosage.

### 5 - Skin sampling

Dorsal skin samples were removed, fixed and stored in formaldehyde (Roti^{®}-Histofix 4%, Carl Roth, Karlsruhe, Allemagne) until histopathological analysis.

The animals were killed by an excessive dose of anaesthetics after the blood and skin samplings.

### 6 - Pro-inflammatory cytokines dosage

The levels of pro-inflammatory cytokines IL-1β, IL-6 et TNF-α were measured simultaneously in duplicate by the Mouse 3-plex A panel Bio-Rad kit (Ref. 171-F11080, Marnes-la-Coquette, France) in the serum samples after thawing.

### 7 - Statistics

Kruskal-Wallis test followed possibly by the Mann-Whitney test was used to compare the different studied variables of the treated groups by comparison with the groups "Control" and "TPA+Control". Significativity threshold was settled at P < 0.05.

Statistics were carried out with the Statview 5 software (SAS, Institute Inc., USA).

### RESULTS

### 1 - Animal weight progress

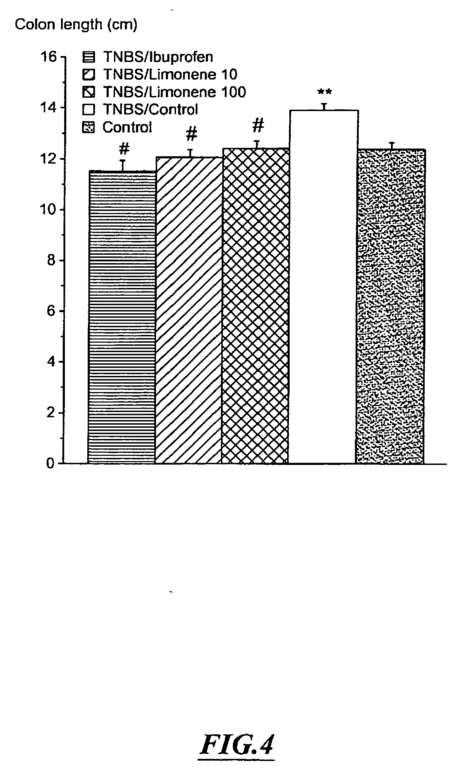

Figure 4 shows the average weight progress of the mice of the 4 groups during the assay.

Between J₋₁ and J₁₀, the average weight of the mice did not show a significant difference between the different groups but a tendency to heterogeneity (H_{(ddl=3)}= 6.73, p = 0.081).

### 2 - Macroscopic scores of skin inflammation

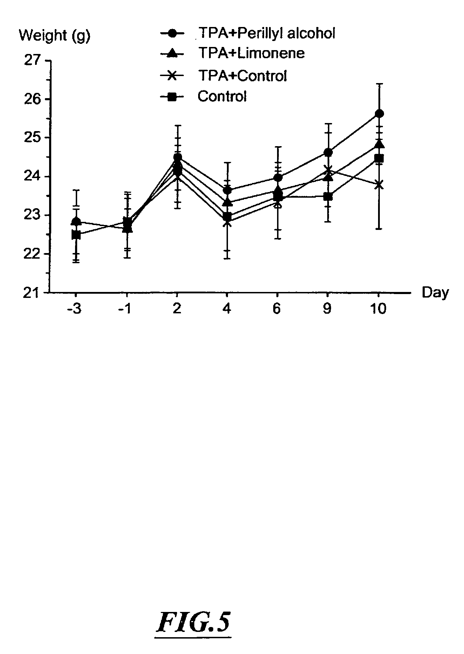

Figure 5 shows the average macroscopic scores of skin inflammation.

The average macroscopic scores of skin inflammation were significantly different between the different groups (H_{(ddl=3)}= 19.83, p = 0.0002).

The average macroscopic scores of skin inflammation of the groups TPA+control, TPA+Limonene and TPA+Perillyl alcohol were significantly higher than the one of the control group (z = 3.00, p = 0.0027 ; z = 3.11, p = 0.0019 ; z = 3.14, p = 0.0017, respectively).

The average macroscopic scores of skin inflammation of the groups TPA+Limonene and TPA+Perillyl alcohol were significantly lower than the one of the group TPA+control (z = 2.62, p = 0.0089 ; z = 2.82, p = 0.0049, respectively). The average macroscopic score of skin inflammation of the group TPA+Perillyl alcohol was significantly lower than the one of the group TPA+Limonene (z = 2.07, p = 0.039).

### 3 - Histopathological analysis of the skin samples

The histopathological analysis of the skin samples removed from the mice of the different treatment groups showed the following results:
- control group: skins were standard,
- TPA+control group: skins were clearly inflammatory; showed non-specific inflammatory skin lesions with epidermis ulceration and with an inflammatory infiltrate often marked,
- TPA+Limonene group: skins were inflammatory; showed non-specific inflammatory skin lesions without epidermis ulceration and with an inflammatory infiltrate more or less marked,
- TPA+Perillyl alcohol group: skins were little inflammatory; showed non-specific inflammatory skin lesions without epidermis ulceration and with an inflammatory rarely marked.

### 4 - Microscopic scores of skin inflammation

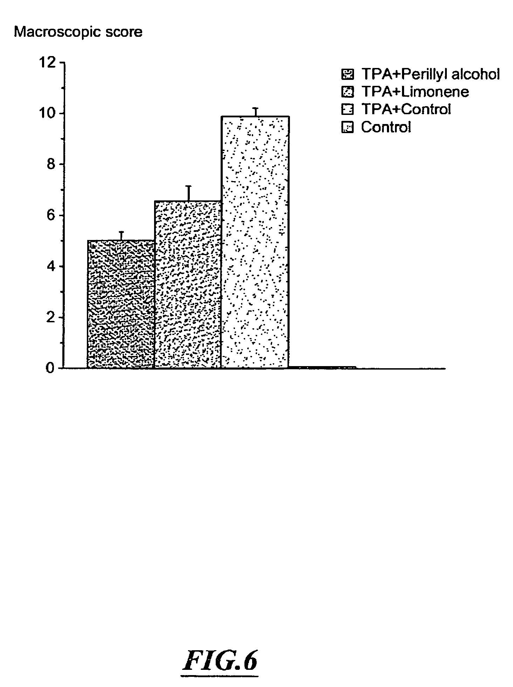

Figure 6 shows the average microscopic scores of skin inflammation of the different treatment groups.

The average microscopic scores of skin inflammation were significantly different between the different groups (H_{(ddl=3)}= 17.49, p = 0.0006).

The average microscopic scores of skin inflammation of the groups TPA+control, TPA+Limonene and TPA+Perillyl alcohol were significantly higher than the one of the control group (z = 2.91, p = 0.0036 in every case).

The average microscopic scores of skin inflammation of the groups TPA+Limonene and TPA+Perillyl alcohol were significantly lower than the one of the group TPA+control (z = 2.10, p = 0.036 ; z = 2.49, p = 0.013, respectively). The average microscopic scores of skin inflammation of the groups TPA+Perillyl alcohol and TPA+Limonene were not significantly different (z = 0.97, p = 0.33).

### 5 - Pro-inflammatory cytokines dosages

### 5.1. IL-1β

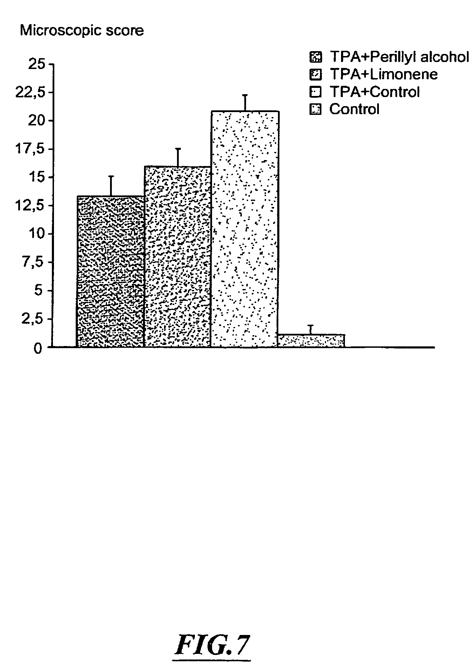

Figure 7 shows the average serum levels of IL-1β of the different treatment groups.

The average serum levels of IL-1β of the different treatment groups were significantly different (H_{(ddl=3)}= 15.62, p = 0.0014) (Table 3).

The average serum levels of IL-1β of the TPA+control, TPA+Limonene and TPA+Perillyl alcohol groups were significantly higher than the one of the control group (z = 3.08, p = 0.0021 in every case).

The average serum level of IL-1β of the TPA+Perillyl alcohol group showed a tendency to be significantly lower than the one of the TPA+control group (z = 1.92, p = 0.055).

No significant difference was shown between the average serum levels of IL-1β of the TPA+Limonene and TPA+Perillyl alcohol groups.

**Table 3**

| **Average serum levels of IL-1β (pg/ml) of the mice of the different treatment groups (Mean ± ESM)** | | | | |
|---|---|---|---|---|
| **Group** | **Control** | **TPA + Control** | **TPA + Limonene** | **TPA + Perillyl alcohol** |
| **Serum levels of IL-1β** | **0.0 ± 0.0** | **9.3 ± 2.9** | **6.5 ± 1.8** | **5.3 ± 1.7** |

### 5.2. IL-6

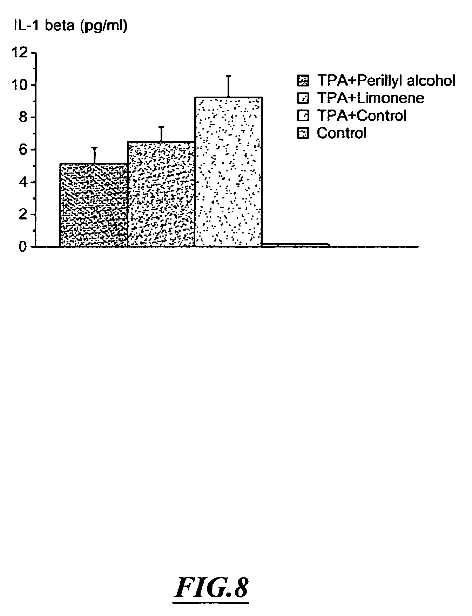

Figure 8 shows the average serum levels of IL-6 of the different treatment groups.

The average serum levels of IL-6 of the different treatment groups were significantly different (H_{(ddl=3)}= 16.42, p = 0.0009) (Table 4).

The average serum levels of IL-6 of the TPA+control, TPA+Limonene and TPA+Perillyl alcohol groups were significantly higher than the one of the control group (z = 3.08, p = 0.0021 in every case).

The average serum level of IL-6 of the TPA+Perillyl alcohol group was significantly lower than the one of the TPA+control group (z = 2.08, p = 0.037).

The average serum level of IL-6 of the TPA+Limonene group showed a tendency to be significantly lower than the one of the TPA+control group (z = 1.76, p = 0.078).

No significant difference was shown between the average serum levels of IL-6 of the TPA+Limonene and TPA+Perillyl alcohol groups.

**Table 4**

| **Average serum levels of IL-6 (pg/ml) of the different treatment groups (Mean ± ESM)** | | | | |
|---|---|---|---|---|
| **Group** | **Control** | **TPA + Control** | **TPA + Limonene** | **TPA + Perillyl alcohol** |
| **Serum levels of IL-6** | **0.0±0.0** | **22.0 ± 5.5** | **15.3 ± 3.0** | **13.0 ± 3.8** |

### 5.3. TNF-α

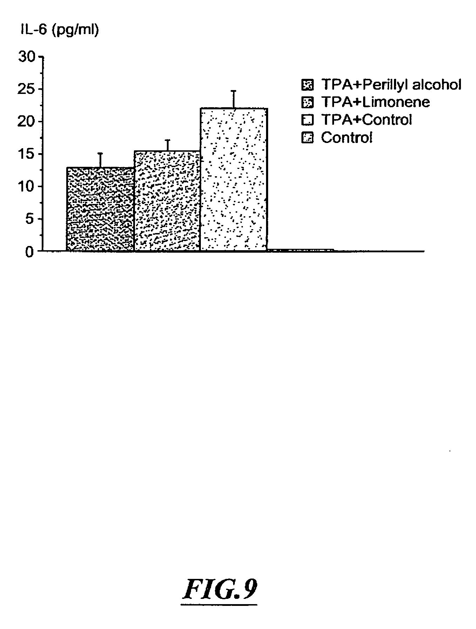

Figure 9 shows the average serum levels of TNF-α of the different treatment groups.

The average serum levels of TNF-α of the different treatment groups were significantly different (H_{(ddl=3)}= 17.08, p = 0.0007) (Table 5).

The average serum levels of TNF-α of the TPA+control, TPA+Limonene and TPA+Perillyl alcohol groups were significantly higher than the one of the control group (z = 3.08, p = 0.0021 in every case).

The average serum level of TNF-α of the TPA+Perillyl alcohol group was significantly lower than the one of the TPA+control group (z = 2.24, p = 0.025). The average serum level of TNF-α of the TPA+Limonene group showed a tendency to be significantly lower than the one of the TPA+control group (z = 1.76, p = 0.078).

No significant difference was shown between the average serum levels of TNF-α of the TPA+Limonene and TPA+Perillyl alcohol groups.

**Table 5**

| **Average serum levels of TNF-α (pg/ml) of the different treatment groups (Mean ± ESM)** | | | | |
|---|---|---|---|---|
| **Group** | **Control** | **TPA + Control** | **TPA + Limonene** | **TPA + Perillyl alcohol** |
| **Serum levels of TNF-α** | **0.0 ± 0.0** | **131.9 ± 18.9** | **103.2 ± 18.0** | **80.6 ± 24.7** |

### 6 - Conclusion

Limonene and Perillyl alcohol, topically administered in preventive treatment during 3 days at 10 mg/kg/day before induction of skin inflammation, showed a significant effect on skin inflammation by reducing the inflammation degree (macroscopic and microscopic scores) and by reducing the pro-inflammatory cytokines secretion (IL-1β, IL-6 et TNF-α).

### Example 3 : Effects of limonene and perillyl alcohol on healing

### MATERIAL AND METHODS

### 1 - Animals

6 female mice hairless Skh-1 with an average weight of 20-25 g were used.

The 6 mice were marked and shared in 3 groups of 2 :
- "control" group = scarification on each flank and daily topical treatment during 8 days with carrier;
- "Limonene" group = scarification on each flank and daily topical treatment during 8 days with limonene 10 mg/kg/day;
- " Perillyl alcohol" group = scarification on each flank and daily topical treatment during 8 days with perillyl alcohol 10 mg/kg/day;

### 2 - Scarification

Each mouse was submitted to a scarification of the skin on each flank.

### 3 -Assayed products and treatment conditions

Limonene ((R)-(+)-Limonene, MW = 136.23, purity = 97%) from SIGMA-ALDRICH (Saint-Quentin Fallavier, France) was used and stored according to SIGMA-FLUKA instructions.

Perillyl alcohol ((S)-4-Isopropenyl-1-cyclohexenylmethanol ; (S)-p-Mentha-1,8-dien-7-ol, MW = 152.23, purity = 98%) from SIGMA-ALDRICH (Saint-Quentin Fallavier, France) was used and stored according to SIGMA-FLUKA instructions.

Limonene and Perillyl alcohol were prepared in maize oil extemporarily every day of treatment.

The daily topical treatment begun 1 hour before the scarification and was continued 8 days after the scarification.

### RESULTS

Daily observations and photographs were performed in order to follow the healing process.

The wound healing process gave better results with daily topical application of a maize oil solution of perillyl alcohol in comparison with limonene and much better results in comparison with corn oil alone.

Fewer marks of healing were also observed at the sites of scarification, at the skin surface and under the skin surface.

### REFERENCES

- Crowell PL et al., "Human metabolism of the experimental cancer therapeutic agent d-limonene", Cancer Chemother Pharmacol. 1994;35(1):31-7;
- Dong Y-L, Fleming RYD, Yan TZ, Herndon DN, Waymack JP. Effect of ibuprofen on the inflammatory response to surgical wounds. Journal of Trauma. 1993; 35(3):340-343;
- Dvivedi S, Tiwari SM, Sharma A. Effect of ibuprofen and diclofenac sodium on experimental wound healing. Indian Journal of Experimental Biology. 1997; 35(11):1243-1245;
- Hardcastle IR et al., "Inhibition of protein prenylation by metabolites of limonene", Biochem Pharmacol. 1999 Apr 1;57(7):801-9;
- Kulick MI, Smith S, Hadler K. Oral ibuprofen: evaluation of its effect on peritendinous adhesions and the breaking strength of a tenorrhaphy. Journal of Hand Surgery. 1986; 11(1):110-120;
- Kumar A, Rao M, Kulkarni DR. Zinc incorporation reverses suppressant effect of ibuprofen on wound healing. Indian Journal of Experimental Biology. 1988; 26(6):483-485.
- Lee KH. Studies on the mechanism of action of salicylate. II. Retardation of wound healing by aspirin. Journal of Pharmaceutical Sciences. 1968; 57(6):1042-1043;
- Rao CM, Kumar A, Kulkarni DR., Effects of enfenamic acid and its zinc salt on wound-heaking. Indian Journal of Physiology and Pharmacology. 1988; 32(1):61-66;
- Stanley P.L., Steiner S;, Havens M., Tramposch K.M., Skin Pharmacol., 4:262-271, 1991;
- Vigushin DM et al., « Phase I and pharmacokinetic study of D-limonene in patients with advanced cancer. Cancer Research Campaign Phase I/II Clinical Trials Committee", Cancer Chemother Pharmacol. 1998;42(2):111-7;

## Claims

1. Cosmetic non-therapeutical use of a monoterpene to increase skin or mucosa tissue repair in an individual, wherein said monoterpene is perillyl alcohol and is used to increase a skin defect repair, said skin defect being selected from the group consisting of cellulitis, scars, sun and UV induced premature skin senescence, unesthetic skin defaults due to the aging process including scars and stains, wrinkles and squamous features, stretch marks, aging marks, sun and UV induced premature skin senescence, and unesthetisms of post-traumatic relapses.

2. The use according to claim 1, to increase biological tissue regeneration.

3. The use according to claim 1 or 2, said individual being a human.

4. A monoterpene for use for preventing or treating a biological tissue injury in an individual, wherein said monoterpene is perillyl alcohol; said tissue injury being selected in the group consisting of burns, cuts, relapses of extreme weather conditions including excessive cold or heat, burning relapses and wounds.

5. A monoterpene for use for preventing or treating a skin disease in an individual, wherein said monoterpene induces tissue repair, said monoterpene being perillyl alcohol and wherein said tissue disease is a skin disease selected from the group consisting of atopic dermatitis, seborrheic keratitis, epidermolysis bullosa acquisita, psoriasis, skin alterations in lupus erythematosus, dermatomyositis, scleroderma, chronic acne, chronic cellulites, pruritus and abnormal or defective scar formation in diabetes.

6. The monoterpene for its use according to any one of claims 4 to 5, wherein said monoterpene is administered topically.

7. The monoterpene for its use according to any one of claims 4 to 6, said individual being a human.

## Patentansprüche

1. Kosmetische, nicht therapeutische Verwendung eines Monoterpens um die Reparatur von Haut- oder Schleimhautgewebe in einem Individuum zu verbessern, wobei das Monoterpen Perillylalkohol ist und verwendet wird, um die Reparatur eines Hautdefekts zu verbessern, wobei der Hautdefekt ausgewählt wird aus der Gruppe bestehend aus Cellulite, Narben, durch Sonne oder UV induzierte vorzeitige Hautalterung, unästhetische Hautfehler aufgrund des Alterungsprozesses einschließlich Narben und Verfärbungen, Falten und schuppenartige Merkmale, Dehnungsstreifen, Alterungszeichen, durch Sonne oder UV induzierte vorzeitige Hautalterung, und unästhetische Merkmale eines posttraumatischen Rezidivs.

2. Die Verwendung gemäß Anspruch 1, um die biologische Geweberegeneration zu verbessern.

3. Die Verwendung gemäß Anspruch 1 oder 2, wobei das Individuum ein Mensch ist.

4. Ein Monoterpen zur Verwendung beim Verhindern oder Behandeln einer Verletzung eines biologischen Gewebes in einem Individuum, wobei das Monoterpen Perillylalkohol ist; und die Verletzung des Gewebes ausgewählt wird aus der Gruppe bestehend aus Verbrennungen, Schnitten, Rezidive durch extreme Wetterbedingungen, einschließlich exzessiver Kälte oder Hitze, Verbrennungsrezidive und Wunden.

5. Ein Monoterpen zur Verwendung beim Verhindern oder Behandeln einer Hauterkrankung in einem Individuum, wobei das Monoterpen Gewebsreparatur induziert und wobei das Monoterpen Perillylalkohol ist und wobei die Hauterkrankung eine Hauterkrankung ist, die ausgewählt wird aus der Gruppe bestehend aus atopischer Dermatitis, seborrhoische Keratitis, Epidermolysis bullosa acquisita, Psoriasis, Hautveränderungen bei Lupus erythematosus, Dermatomyositis, Sklerodermie, chronische Akne, chronische Cellulite, Pruritus und abnormale oder fehlerhafte Narbenbildung bei Diabetes.

6. Das Monoterpen zur Verwendung gemäß einem der Ansprüche 4 bis 5, wobei das Monoterpen topisch verabreicht wird.

7. Das Monoterpen zur Verwendung gemäß einem der Ansprüche 4 bis 6, wobei das Individuum ein Mensch ist.

## Revendications

1. Utilisation cosmétique non thérapeutique d'un monoterpène pour augmenter la réparation de la peau ou de tissus muqueux chez un individu, dans laquelle ledit monoterpène est l'alcool périllique et est utilisé pour augmenter la réparation d'un défaut cutané, ledit défaut cutané étant sélectionné dans le groupe consistant en la cellulite, les cicatrices, la sénescence cutanée prématurée induite par le soleil et les UV, les défauts cutanés inesthétiques dus au processus de vieillissement comprenant les cicatrices et les taches, les rides et les caractéristiques squameuses, les vergetures, les marques du vieillissement, la sénescence cutanée prématurée induite par le soleil et les UV, et les inesthétismes de rechutes post-traumatiques.

2. Utilisation selon la revendication 1, pour augmenter la régénération d'un tissu biologique.

3. Utilisation selon la revendication 1 ou 2, ledit individu étant un humain.

4. Monoterpène pour son utilisation dans la prévention ou le traitement d'une lésion d'un tissu biologique chez un individu, ledit monoterpène étant l'alcool périllique ; ladite lésion de tissu étant sélectionnée dans le groupe consistant en les brûlures, les coupures, les rechutes de conditions météorologiques extrêmes incluant un froid ou une chaleur excessifs, les rechutes de brûlures et les plaies.

5. Monoterpène pour son utilisation dans la prévention ou le traitement d'une maladie de la peau chez un individu, où ledit monoterpène induit une réparation tissulaire, ledit monoterpène étant l'alcool périllique et où ladite maladie tissulaire est une maladie de la peau sélectionnée dans le groupe consistant en la dermatite atopique, la kératose séborrhéique, l'épidermolyse bulleuse acquise, le psoriasis, les altérations cutanées dans un contexte de lupus érythémateux, la dermatomyosite, la sclérodermie, l'acné chronique, la cellulite chronique, le prurit et une formation de cicatrices anormale ou déficiente dans un contexte de diabète.

6. Monoterpène pour son utilisation selon l'une quelconque des revendications 4 à 5, où ledit monoterpène est administré par voie topique.

7. Monoterpène pour son utilisation selon l'une quelconque des revendications 4 à 6, ledit individu étant un humain.
